# EUROPÄISCHE PATENTSCHRIFT

(11) **EP 1 723 075 B1**
(45) Veröffentlichungstag und Bekanntmachung des Hinweises auf die Patenterteilung: **17.10.2012**
(21) Anmeldenummer: 05707275.3
(22) Anmeldetag: 09.02.2005
(51) Int. Cl.: C01B 31/28

(54) **HERSTELLUNG VON CARBONYLFLUORID**
PRODUCTION OF CARBONYL FLUORIDE
PRODUCTION DE FLUORURE DE CARBONYLE

(30) Priorität: 08.03.2004 EP 04005421
(43) Veröffentlichungstag der Anmeldung: 22.11.2006
(73) Patentinhaber: Solvay Fluor und Derivate GmbH, 30173 Hannover (DE)
(72) Erfinder: BRAUN, Max, 30900 Wedemark (DE); EICHER, Johannes, 31319 Sehnde (DE)
(74) Vertreter: Jacques, Philippe
(86) Internationale Anmeldenummer: PCT/EP2005/001281
(87) Internationale Veröffentlichungsnummer: WO 2005/085129

(56) Entgegenhaltungen:
- DATABASE CHEMABS [Online] CHEMICAL ABSTRACTS SERVICE, COLUMBUS, OHIO, US; KUZ'MENKO, V. A.: "Reactions of carbon difluoride and oxygen. Chlorine catalysis" XP002296381 gefunden im STN Database accession no. 112:14054 & ZHURNAL FIZICHESKOI KHIMII , 63(7), 1911-12 CODEN: ZFKHA9; ISSN: 0044-4537, 1989,
- NEVE DE MEVERGNIES M: "Vibrational photochemistry of fluoroform induced by CO2 laser pulses" INFRARED PHYSICS UK, Bd. 25, Nr. 1-2, Februar 1985 (1985-02), Seiten 175-189, XP002354332 ISSN: 0020-0891
- KOJIMA, MASANOBU ET AL: "Photolysis of carbon dioxide with 158 nm (F2) laser. Reactivity of atomic oxygen(1D) with methane, trifluoromethane, and 1,1,1-trifluoroethane" CHEMISTRY LETTERS , (7), 1309-12 CODEN: CMLTAG; ISSN: 0366-7022, 1992, XP008055546
- ATKINSON R ET AL: "TROPHOSPHERIC AND STRATOSPHERIC SINKS FOR HALOCARBONS: PHOTOOXIDATION, O(1D) ATOM, AND OH RADICAL REACTIONS" JOURNAL OF GEOGRAPHICAL RESEARCH, RICHMOND, VA, US, Bd. 81, Nr. 33, 20. November 1976 (1976-11-20), Seiten 5765-5770, XP008032999 ISSN: 0148-0227
- EDNEY E O ET AL: "CHLORINE INITIATED PHOTOOXIDATION STUDIES OF HYDROCHLOROFLUOROCARBONS (HCFCS) AND HYDROFLUOROCARBONS (HFCS): RESULTS FOR HCFC-22 (CHCLF2); HFC-41 (CH3F); HCFC-124 NCCIFHCF3); HFC-125 (CF3CHF2); HFC-134A (CF3CH2F); HCFC-142B (CCLF2CH3); AND HFC-152A (CHF2" INTERNATIONAL JOURNAL OF CHEMICAL KINETICS, WILEY, NEW YORK, NY, US, Bd. 24, Nr. 12, 1992, Seiten 1067-1081, XP008032981 ISSN: 0538-8066 in der Anmeldung erwähnt
- BROWNSWORD RICHARD A ET AL: "Photodissociation dynamics of CHF2Cl after photoexcitation at the Lyman-alpha wavelength (121.6 nm)" J PHYS CHEM A; JOURNAL OF PHYSICAL CHEMISTRY A MOLECULES FEB 6 1997 ACS, WASHINGTON, DC, USA, Bd. 101, Nr. 6, 6. Februar 1997 (1997-02-06), Seiten 995-999, XP002296380
- DATABASE CHEMABS [Online] CHEMICAL ABSTRACTS SERVICE, COLUMBUS, OHIO, US; ZHONG, JINXIAN ET AL: "The photolysis characteristics of HCFC-22 in presence of hydrogen peroxide" XP002296382 gefunden im STN Database accession no. 126:218375 & HUANJING KEXUE , 17(3), 54-56 CODEN: HCKHDV; ISSN: 0250-3301, 1996,
- DATABASE CHEMABS [Online] CHEMICAL ABSTRACTS SERVICE, COLUMBUS, OHIO, US; KOMAROV, V. S. ET AL: "Reaction of ozone with halogen-substituted saturated hydrocarbons" XP002296383 gefunden im STN Database accession no. 93:132037 & KINETIKA I KATALIZ , 21(2), 519-20 CODEN: KNKTA4; ISSN: 0453-8811, 1980,

## Beschreibung

Die Erfindung bezieht sich auf die Herstellung von Carbonylfluorid (Fluorphosgen) durch photochemische Oxidation.

Carbonylfluorid ist als neues Ätzgas für die Reinigung von CVD-Reaktoren vorgeschlagen worden. Die technische Herstellung ist durch Erhitzen eines Monohalodifluormethans möglich, siehe EP-A-0 310255. In wissenschaftlichen Publikationen ist auch die photochemische Oxidation von Chlordifluormethan in Anwesenheit von Chlor beschrieben worden, siehe E. O. Edney und D. J. Driscoll, Int. Journal of Chemical Kinetics, Vol. 24(1992), Seiten 1067 bis 1081. Der Gehalt an HCFC-22 in der Bestrahlungszelle lag im ppm-Bereich, der Druck betrug 700 Torr. Ziel war es, Erkenntnisse über die troposphärische Zersetzung verschiedener Halogenkohlenwasserstoffe zu erhalten.

In der Publikation von V. A. Kuzmenko in Zhurnal Fizicheskoi Khimii 63 (1989), Seiten 1911 und 1912 wurde ein IR-Laser zur Bestrahlung eines Gemisches eingesetzt, das HCFC-22, Sauerstoff und Chlor enthielt.

R. Atkinson, G. M. Breuer, J. N. Pitts, jr. und H. L. Sandoval beschreiben im Journal of Geophysical Research 81 (1976), Seiten 5765 bis 5770 die Photooxidation von HCFC-22 im Hinblick auf das Verhalten dieser Verbindung in der Troposphäre und Stratosphäre. Die Reaktion wurde bei niedrigem Druck und in Anwesenheit von Stickstoffdioxid und ggf. N₂O für eine Zeitdauer von 20 bis 80 Stunden durchgeführt.

Andere Autoren haben die Photooxidation von HCFC-22 in Anwesenheit von Wasserstoffperoxid beschrieben.

Aufgabe der vorliegenden Erfindung war es, ein technisch vorteilhaft durchführbares Verfahren zur Herstellung von Carbonylfluorid, C(O)F₂, anzugeben. Diese Aufgabe wird durch das Verfahren der vorliegenden Erfindung gelöst.
Das erfindungsgemäße Verfahren sieht die Herstellung von C(O)F₂ durch Photooxidation von CHClF₂ oder CHF₃ mit Sauerstoff vor. Dabei werden vorzugsweise keine Laser-Strahler als Strahlungsquelle verwendet, sondern es wird bevorzugt Licht eingestrahlt, das nicht aus einer einzigen Wellenlänge besteht, sondern einen Spektralbereich aufweist, der mindestens 50 nm umfasst (d.h. der Lichtanteil mit niedrigster Wellenlänge und der Lichtanteil mit der höchsten Wellenlänge liegen mindestens 50 nm auseinander). Vorzugsweise liegt mindestens ein Teil der Strahlung dabei im Bereich von 280 nm bis hin zum langwelligen Ende des sichtbaren Lichtes, d.h. bis hin zu etwa 750 nm. Das besagt aber nicht, dass Strahlung über den gesamten Bereich kontinuierlich abgegeben werden muss. Dabei wird der Begriff "Licht" nicht einschränkend auf "sichtbares Licht" benutzt; er umfasst auch Strahlung außerhalb des Bereichs des sichtbaren Lichts.

Die Verwendung von CHClF₂ (HCFC-22) ist bevorzugt und dient zur weiteren Erläuterung der Erfindung.

Der Druck im Reaktor entspricht bevorzugt mindestens dem Umgebungsdruck, also 1 bar (abs.). Er kann auch darüber liegen. Der Druck liegt bevorzugt im Bereich von 1 bar (abs.) bis 11 bar (abs.). Die Temperatur liegt bevorzugt im Bereich von 20 bis 300 °C, besonders im Bereich von 30 bis 300 °C, insbesondere im Bereich von 30 bis 90 °C und ganz besonders im Bereich von 50 bis 90 °C. Vorteilhaft wählt man die Bedingungen hinsichtlich Druck und Temperatur derart, dass das Reaktionsgemisch gasförmig bleibt.
Ganz besonders bevorzugt arbeitet man drucklos. Der Begriff "drucklos" bedeutet im Rahmen der vorliegenden Erfindung, dass auf die Reaktionsmischung außer dem Umgebungsdruck (d.h. etwa 1 bar), dem Förderdruck der Halogenkohlenstoff-Ausgangsverbindung sowie des Sauerstoffgases (bzw. des sauerstoffhaltigen Gases, man kann z.B. Luft oder Sauerstoff/Inertgas-Gemische einsetzen) und des gegebenenfalls eingesetzten Chlors sowie dem sich gegebenenfalls ausbildenden Druck durch bei der Reaktion entstehendes Chlorwasserstoffgas kein zusätzlicher Druck einwirkt. Der Gesamtdruck im Reaktor ist dann zweckmäßig kleiner als 2 bar absolut, je nach Förderdruck sogar kleiner als 1,5 bar absolut, aber größer als der Umgebungsdruck.

Anders als im Stand der Technik liegt das HCFC-22 in der vorliegenden Erfindung nicht im ppm-Bereich, sondern in signifikanter Menge im Reaktor vor. So beträgt sein Anteil im Reaktionsgemisch bevorzugt mindestens 5 mol.-%, vorzugsweise mindestens 10 mol-%.
Das Verfahren kann batchweise oder bevorzugt kontinuierlich durchgeführt werden. Vorzugsweise geht man so vor, dass man kontinuierlich Ausgangsmaterial (die entsprechende Ausgangsverbindung, ein Sauerstoff enthaltendes Gas wie Luft oder reinen Sauerstoff und gegebenenfalls Chlor) in eine Durchflussapparatur einspeist und entsprechend der eingespeisten Menge kontinuierlich Reaktionsprodukt bzw. Reaktionsgemisch abzieht. Die durchschnittliche Verweilzeit im Reaktionsgefäß liegt vorteilhaft zwischen 0,01 und 30 Minuten, bevorzugt zwischen 0,1 bis 3 min, besonders bevorzugt zwischen 0,3 und 1,5 Minuten. Die optimale durchschnittliche Verweilzeit, die u.a. von der Art der Lampen, der Strahlungsleistung der Lampen und von geometrischen Parametern der Bestrahlungsapparatur abhängig ist, kann man durch einfache Handversuche und Analyse des Produktstroms, beispielsweise durch Gaschromatographie, ermitteln. Vorteilhaft kann es auch sein, die Reaktionsmischung beispielsweise durch geeignete Einbauten im Reaktor gut zu verwirbeln. Die optimale Verweilzeit bei batchweiser Durchführung kann in gleicher Weise ermittelt werden.

Das Verfahren kann in zwei bevorzugten Ausführungsformen durchgeführt werden, nämlich in Abwesenheit von Chlor, oder, bevorzugt, in Anwesenheit von Chlor als Initiator. Bei Anwesenheit von Chlor als Initiator ist es wünschenswert, einen bestimmten Wellenbereich, nämlich denjenigen unterhalb von 280 nm, nicht auf die Reaktionsmischung einwirken zu lassen. Beide Ausführungsformen werden im Folgenden erläutert.

Eine Ausführungsform sieht also die Photooxidation in Abwesenheit von Chlor oder anderen Radikalinitiatoren oder Aktivatoren vor. Beispielsweise kann die Bestrahlung durch Quarzglas hindurch vorgenommen werden; andere Bauteile des Reaktors, die nicht zwischen Lichtquelle und Reaktionsgemisch angeordnet sind, können natürlich aus beliebigen Bauteilen, z.B. auch aus Borsilikat-Glas sein (welches bestimmte Strahlungsanteile filtert, siehe unten). Als Strahler eignen sich übliche Strahler, die beispielsweise Strahlung im Bereich von 250 bis 400 nm oder sogar bis 600 nm abgeben (das Spektrum kann auch über die untere oder obere Grenze hinausgehen, beispielsweise bis zur Grenze des sichtbaren Lichts, etwa 750 nm). Bei der Abwesenheit von Chlor ist es unbedenklich, wenn Licht unterhalb von 280 nm auf die Reaktionsmischung einwirkt.

Eine weitere, bevorzugte Ausführungsform sieht die Bestrahlung in Anwesenheit von elementarem Chlor unter Bestrahlung mit Licht einer Wellenlänge von ≥ 280 nm vor, wobei pro Mol CHClF₂ maximal 0,5 Mol elementares Chlor im Reaktionsgemisch enthalten sind. Bevorzugt werden pro Mol CHClF₂ 0,01 bis 0,50 mol Chlor, vorzugsweise 0,03 bis 0,25 mol, insbesondere 0,05 bis 0,20 mol elementares Chlor eingesetzt.

Wasserstoffperoxid, Ozon oder Stickstoffoxide wie N₂O oder NO₂ werden bevorzugt nicht dem Reaktionsgemisch zugesetzt.

Umsatzrate, Ausbeute und Selektivität sind besonders hoch, wenn man HCFC-22 und Sauerstoff in Anwesenheit von elementarem Chlor umsetzt und eine aktivierende Bestrahlung mit Licht einer Wellenlänge λ ≥ 280 nm vornimmt. Frequenzen einer Wellenlänge unterhalb von 280 nm sind dann im Wesentlichen aus dem Frequenzspektrum ausgeblendet. Dies kann man dadurch bewirken, dass man Bestrahlungslampen verwendet, die nur Licht einer Wellenlänge oberhalb oder bei 280 nm abstrahlen, und/oder man verwendet Mittel, die Frequenzen unterhalb von 280 nm aus dem abgestrahlten Licht ausblenden. Beispielsweise kann man durch Glas bestrahlen, welches nur für Licht einer Wellenlänge von 280 nm oder darüber durchlässig ist, also den kürzerwelligen Strahlungsanteil herausfiltert. Gut geeignet dafür sind beispielsweise Borosilikat-Gläser. Geeignete Gläser enthalten beispielsweise 7 bis 13 % B₂O₃, 70 bis 80 % SiO₂, ferner 2 bis 7 % Al₂O₃ und 4 bis 8 % Na₂O + K₂O sowie 0 bis 5% Erdalkalimetalloxide (jeweils Gew.-%). Bekannte Marken für Borosilikat-Gläser sind Duran, Pyrex und Solidex.

Zur Bestrahlung sind besonders gut Bestrahlungslampen geeignet, die nur (UV)Licht einer Wellenlänge ≥ 280 nm abstrahlen. Insbesondere Leuchtstoff-Röhren (z.B. von der Firma Philips) sind sehr gut geeignet. Mit derartigen Lampen kann man die Bestrahlung durch Quarzglas, aber auch durch die vorstehend beschriebenen, den kürzerwelligen Bestrahlungsanteil herausfilternde Gläser vornehmen. Voraussetzung ist natürlich, dass die verwendeten Lampen oder Röhren auch im Absorptionsbereich des elementaren Chlors emittieren. Neben den besonders gut geeigneten Leuchtstoffröhren kann man auch beispielsweise Bestrahlungslampen (z.B. Quecksilber-Mittel- oder Hochdruckstrahler) verwenden; etwaige Linien im Bereich unterhalb von 280 nm werden dann herausgefiltert, beispielsweise indem man durch ein Glas bestrahlt, das nur für Licht einer Wellenlänge bei und oberhalb von 280 nm durchlässig ist. Verwendbare Gläser sind weiter oben beschrieben. Gut geeignet zur Bestrahlung sind auch Lampen, z.B. Quecksilber-Hochdrucklampen, die aufgrund eines Dotierungsmittels überwiegend oder nur im bevorzugten Wellenbereich bei und oberhalb von 280 nm abstrahlen. Quecksilber-Hochdruckstrahler beispielsweise weisen eine recht intensive Bande im Bereich von 254 nm auf, die, wie oben beschrieben wird, beispielsweise durch Borosilikat-Glas herausgefiltert werden kann. Bei durch Metalljodide dotierten Quecksilber-Hochdruckstrahlern ist diese Linie stark unterdrückt. Überraschend ist die oft überproportionale Erhöhung der Umsatzrate bei Verwendung solcher dotierten Strahler. Besonders gut geeignet sind Quecksilber-Hochdruckstrahler, die mit Galliumjodid, insbesondere Thalliumjodid oder Cadmiumjodid dotiert sind. Auch bei Verwendung solcher dotierter Strahlungslampen filtert man vorteilhaft den kürzerwelligen Strahlungsanteil mit λ < 280 nm heraus, beispielsweise indem man in Borosilikat-Glas arbeitet.

Das Molverhältnis zwischen der Ausgangsverbindung und Sauerstoff kann in einem weiten Bereich schwanken, zweckmäßig setzt man jedoch mindestens 0,4 Mol Sauerstoff pro Mol Ausgangsverbindung ein. Der Sauerstoff kann auch im Überschuß eingesetzt werden. Besonders gute Ergebnisse werden erzielt, wenn das Molverhältnis zwischen der Ausgangsverbindung und dem Sauerstoff im Bereich von 1:0,4 bis 1:5, vorzugsweise 1:0,4 bis 1:1, insbesondere 1:0,4 bis 1:0,9 liegt. Der Sauerstoff kann wie gesagt in Form von Luft eingesetzt werden. Bevorzugt setzt man den Sauerstoff in Form eines O₂/inertgas-Gemisches, insbesondere aber als reinen Sauerstoff ein. In Bezug auf die Produktreinheit ist es wünschenswert, dass möglichst wenig Wasser bei der Umsetzung vorhanden ist (beispielsweise weniger als 30 ppm). Gewünschtenfalls kann man die Reaktanten in bekannter Weise von mitgeschlepptem Wasser befreien, z.B. mittels Molekularsieb.

Der Vorteil des erfindungsgemäßen Verfahrens ist die hohe Selektivität und Ausbeute.

Die folgenden Beispiele erläutern die Erfindung, ohne sie einzuschränken.

### Beispiel 1: Herstellung von Fluorphosgen (COF₂) durch photochemische Reaktion

Reaktionsgleichung: CF₂HCl + ½ O₂ → COF₂ + HCl
Ansatzgröße: siehe jeweiliger Versuch

### Versuchsdurchführung und Aufbau:

Als Reaktionsraum diente ein aus Duran-Glas gefertigter Reaktor mit einem Füllvolumen von maximal 580 ml, welcher einen Kühlfinger (Duran) und einen Lampenschacht (Quarzglas) aufwies. Die Gaseinleitung erfolgte über eine Glasfritte, die sich am Reaktorboden befand. Der Quecksilberdampf-Hochdruckstrahler wurde mit Pressluft gekühlt.

Zu Versuchsbeginn wurde zuerst die Pressluftkühlung aufgedreht und dann die Lampe gezündet. Nach ca. 10 min hat der Strahler seine Leistung (500 oder 700Watt) erreicht. Es wurde jetzt die Einleitung der Gase begonnen. Zunächst wurde die Einleitung von HCFC-22 (R 22) gestartet, dann die Einleitung des Chlors, schließlich auch noch die Einleitung des Sauerstoffs, so dass alle drei Reaktanten in den Reaktor eingespeist wurden.

Alle Gase wurden dann in einem bestimmten Verhältnis zugleich dosiert und durch den Reaktorraum geleitet. ("Wenig Chlor" bedeutet etwa 0,12 mol/h Chlor pro 1 mol/h CHF₂Cl). Der entstehende Produktgasstrom wurde durch eine Waschflasche (gefüllt mit ca. 5%iger H₂O₂- Lösung) geleitet, um das überschüssige Chlor aufzufangen und in HCl umzuwandeln. Die Proben des Produktgasstroms wurden vor der Waschflasche entnommen.

### Versuch 1:

| | | | | | | | |
|---|---|---|---|---|---|---|---|
| Ansatz: | 0,5 mol R22/h | | | | | | |
| | 0,5 mol O₂/ | | | | | | |
| | wenig Cl₂ | | | | | | |
| Durchführung: | Lampenleistung bei 700 Watt | | | | | | |

| Probenahme und Uhrzeit (Beginn 7:10) | R22 (in g) | R22 mol/h | Cl2 (in g) | Cl2 mol/h | O2 (in g) | O2 mol/h | Verweilzeit im Reaktor (in min) |
|---|---|---|---|---|---|---|---|
| 07:30 | 17,2 | 0,6 | 2 | 0,08 | 4,8 | 0,5 | 1,23 |
| 07:45 | 37,7 | 0,9 | 2,7 | 0,04 | 9,6 | 0,6 | 0,94 |
| 08:10 | 54,6 | 0,5 | 4,8 | 0,07 | 14,6 | 0,4 | 1,49 |

Analysenauswertung der Gasproben (alle Analysen ohne Luft berechnet):

### Probennahme:

| | | | |
|---|---|---|---|
| um 7.45 Uhr: | 45,1% COF₂ | um 8.10 Uhr: | 24,5% COF₂ |
| | 44,2% HCl | | 23,7% HCl |
| | 8,6% CO₂ | | 10,9% CO₂ |
| | 1,8% R12 | | 3,9% R12 |
| | 0,3% H₂O | | 37,0% R22 |

### Versuch 2:

| | | | | | | | |
|---|---|---|---|---|---|---|---|
| Ansatz: | 0,5 mol R22/h | | | | | | |
| | 0,5 mol O₂/h | | | | | | |
| | wenig Cl₂ | | | | | | |
| | | | | | | | |
| Durchführung: | Lampenleistung bei 500 Watt | | | | | | |

| Probenahme und Uhrzeit Beginn 7:30 | R22 (in g) | R22 mol/h | Cl2 (in g) | Cl2 mol/h | O2 (in g) | O2 mol/h | Verweilzeit im Reaktor (in min) |
|---|---|---|---|---|---|---|---|
| 07:50 | 20,7 | 0,7 | 1,8 | 0,08 | 5,8 | 0,5 | 1,13 |
| 08:45 | 80 | 0,6 | 3,8 | 0,03 | 21,7 | 0,5 | 1,28 |
| 09:45 | 130,8 | 0,6 | 11,3 | 0,1 | 38,9 | 0,5 | 1,21 |
| 11:15 | 220,3 | 0,7 | 14,2 | 0,03 | 56,9 | 0,4 | 1,28 |
| 12:00 | 264,5 | 0,7 | 18,3 | 0,08 | 75,8 | 0,8 | 0,92 |
| 13:00 | 303,7 | 0,5 | 22,0 | 0,1 | 84,4 | 0,3 | 1,71 |
| 13:30 | 342,9 | 0,9 | 0 | 0 | 97,9 | 0,8 | 0,85 |

Analysenauswertung: (alle Analysen ohne Luft berechnet, ausgenommen die Probe um 13.30 Uhr):

| | | | |
|---|---|---|---|
| Probennahme: | | | |
| um 7.50 Uhr: | 32,9% COF₂ | 8.45 Uhr | 43,1% COF₂ |
| | 34,3% HCl | | 42,7% HCl |
| | 5,5% CO₂ | | 6,1% CO₂ |
| | 8,6% R12 | | 6,5% R12 |
| | 0,3% H₂O | | 0,8% R22 |
| | 18,4% R22 | | |
| um 9.45 Uhr | 44,6% COF₂ | 11.15 Uhr | 45,6% COF₂ |
| | 41,6% HCl | | 43,9% HCl |
| | 3,1% CO₂ | | 5,7% CO₂ |
| | 6,8% R12 | | 3,6% R12 |
| | 4,0% R22 | | 1,3% R22 |
| um 12.00 Uhr | 44,9% COF₂ | um 13.00 Uhr | 42,0% COF₂ |
| | 40,3% HCl | | 41,8% HCl |
| | 11,8%CO₂ | | 13,9% CO₂ |
| | 2,6% R12 | | 1,7% R12 |
| | 0,3% R22 | | 0,5% H₂O |
| um 13.30 Uhr | 49,3% Luft (O₂) | | |
| | 44,0% R22 | | |
| | 2,2% HCl | | |
| | 2,2% CO₂ | | |
| | 2,0%COF₂ | | |
| | 0,2% H₂O | | |

### Versuch 3:

| | | | | | | | |
|---|---|---|---|---|---|---|---|
| Ansatz: | 0,5 mol R22/h | | | | | | |
| | 0,5 mol O₂/h | | | | | | |
| | wenig Cl₂ | | | | | | |
| Durchführung: | Lampenleistung bei 500 Watt | | | | | | |

| Probenahme und Uhrzeit (Beginn 7:45) | R22 (in g) | R22 mol/h | Cl2 (in g) | Cl2 mol/h | O2 (in g) | O2 mol/h | Verweilzeit im Reaktor (in min) |
|---|---|---|---|---|---|---|---|
| 08:45 | 61,9 | 0,7 | 4,7 | 0,07 | 16,3 | 0,5 | 1,14 |
| 09:45 | 118,8 | 0,7 | 8,6 | 0,06 | 33 | 0,5 | 1,15 |
| 11:15 | 205,7 | 0,7 | 12,5 | 0,04 | 58,8 | 0,5 | 1,17 |
| 11:45 | 242,6 | 0,9 | 12,7 | 0,006 | 65,6 | 0,4 | 1,11 |

Analysenauswertung (alle Analysen ohne Luft berechnet):

| | | | |
|---|---|---|---|
| Probennahme: | | | |
| um 8.45 Uhr: | 43,6% COF₂ | um 9.45 Uhr: | 46,0% COF₂ |
| | 42,3% HCl | | 43,2% HCl |
| | 10,7% CO₂ | | 6,9% CO₂ |
| | 1,7% R12 | | 1,2% R12 |
| | 1,0% R22 | | 2,2% R22 |
| | 0,6% H₂O | | 0,6% H₂O |
| | | | |
| um 11.15 Uhr | 36,7% COF₂ | um 11.45 Uhr | 41,7% COF₂ |
| | 38,4% HCl | | 40,7% HCl |
| | 8,4% CO₂ | | 7,2% CO₂ |
| | 0,9% R12 | | 0,9% R12 |
| | 15,4% R22 | | 9,3% R22 |
| | 0,2% H₂O | | 0,3% H₂O |

### Beispiel 2: Herstellung von Fluorphosgen (COF₂) durch photochemische Reaktion (mit Quarzglaskühlfinger und ohne Cl₂)

### Versuchsdurchführung und Aufbau:

Als Reaktionsraum dient ein aus Duran-Glas gefertigter Reaktor mit einem Füllvolumen von maximal 580ml, welcher einen aus Quarz gefertigten Kühlfinger und einen Lampenschacht (Quarzglas) aufwies. Die Gaseinleitung erfolgte über eine Glasfritte, die sich am Reaktorboden befand. Der Quecksilberdampf- Hochdruckstrahler wurde mit Pressluft gekühlt. Zu Versuchsbeginn wurde zuerst die Pressluftkühlung aufgedreht und dann die Lampe gezündet. Nach ca. 10 min hatte der Strahler seine Leistung erreicht. Zunächst wurde HCFC-22 in den Reaktor eingeleitet und dann der Sauerstoff zugeschaltet.

Die beiden Gase wurden nun in einem bestimmten Verhältnis zugleich dosiert und durch den Reaktorraum geleitet. Der entstehende Produktgasstrom wurde analysiert.

### Versuch 2.1:

| | | | | | |
|---|---|---|---|---|---|
| Ansatz: | 0,5 mol R22/h | | | | |
| | 0,4 mol O₂/h | | | | |
| | | | | | |
| Durchführung: | Lampenleistung bei 500 Watt | | | | |

| Probenahme und Uhrzeit (Beginn 9:00) | R22 (in g) | R22 mol/h | O2 (in g) | O2 mol/h | Verweilzeit im Reaktor (in min) |
|---|---|---|---|---|---|
| 09:30 | 29,5 | 0,68 | 11,5 | 0,70 | 1,05 |
| 10:00 | 43,8 | 0,51 | 19,0 | 0,59 | 1,32 |
| 10:30 | 62,5 | 0,43 | 26,0 | 0,44 | 1,67 |
| 11:00 | 83,6 | 0,49 | 35,0 | 0,56 | 1,38 |
| 11:30 | 102,3 | 0,43 | 40,0 | 0,31 | 1,96 |
| 12:00 | 120,2 | 0,41 | 45,5 | 0,34 | 1,93 |
| 13:00 | 157,1 | 0,43 | 55,5 | 0,31 | 1,96 |
| 13:30 | 176,3 | 0,44 | 61,0 | 0,34 | 1,86 |

### Analysenauswertung:

| | | | |
|---|---|---|---|
| Probennahme: | | | |
| um 9.30 Uhr | 56,2% O₂ | 10.00 Uhr | 38,8% COF₂ |
| | 15,6% COF₂ | | 34,7% HCl |
| | 9,7% HCl | | 7,7% CO₂ |
| | 1,3%CO₂ | | 14,2% R22 |
| | 16,6% R22 | | 0,4% H₂O |
| | 0,4% H₂O | | 3,7% COFCl |
| | 0,24% COFCl | | 0,6% R12 |
| | | | 0,04% COFCl₂ |
| | | | |
| Um 10.30 Uhr | 35,9% COF₂ | um 11.00 Uhr | 35,4% COF₂ |
| | 31,3% HCl | | 32.3% HCl |
| | 6,1% CO₂ | | 7,1% CO₂ |
| | 21,4% R22 | | 18,6% R22 |
| | 0,2% H₂O | | 5,7% COFCI |
| | 4,5% COFCl | | 0,8% R12 |
| | 0,6% R12 | | 0,07% COCl₂ |
| | 0,05% COCl₂ | | |
| | | | |
| um 11.30 Uhr | 33,6% COF₂ | um 12.00 Uhr | 31,2% COF₂ |
| | 33,7% HCl | | 29,9% HCl |
| | 8,1% CO₂ | | 7,9% CO₂ |
| | 18,6% R22 | | 24,4% R22 |
| | 5,7% COFCI | | 5,7% COFCI |
| | 0,7% R12 | | 0,9% R12 |
| | 0,1% COCl₂ | | 0,1% COCl₂ |
| | | | |
| um 13.00 Uhr | 30,9% COF₂ | um 13.30 Uhr | 27,1% COF₂ |
| | 28,0% HCl | | 30,4% HCl |
| | 6,8% CO₂ | | 11,5% CO₂ |
| | 27,3% R22 | | 23,5% R22 |
| | 0,2% H₂O | | 6,4% COFCl |
| | 5,9% COFCI | | 1,0% R12 |
| | 0,7% R12 | | 0,2% COCl₂ |
| | 0,1% COCl₂ | | |

Die Beispiele belegen, dass besonders gute Ausbeute und Umsatz bei der Durchführung in Anwesenheit von Chlor und mit Licht erreicht werden, dessen kürzerwelliger Anteil (λ<280 nm) herausgefiltert ist.

### Beispiel 3: Herstellung von C(O)F₂ mit einem Molverhältnis von HCFC-22 zu O₂ von 1:0,8

Im vorstehend beschriebenen Reaktor mit ca. 580 ml Innenvolumen wurden HCFC-22, O₂ und Cl₂ mit einem Durchsatz von 1,0 mol/h HCFC-22, 0,8 mol/h O₂ und 0,06 mol/h Cl₂ eingespeist, so dass sich eine Verweilzeit von etwa 1 min ergab, und bei 50 °C miteinander umgesetzt.

Eine Wiederholung des Versuches wurde mit einem Durchsatz von 0,8 mol/h HCFC-22, 0,64 mol/h O₂ und 0,05 mol/h Cl₂ vorgenommen.

Bei gutem Umsatz wurde eine Selektivität von ca. 99,0 bis 99,3 % an C(O)F₂ erreicht.

Die Isolierung des Carbonylfluorids kann nach üblichen Methoden erfolgen, beispielsweise durch Tieftemperatur- oder Druckdestillation.

## Patentansprüche

1. Verfahren zur Herstellung von C(O)F₂ durch Photooxidation von CHClF₂ oder CHF₃ mit Sauerstoff, wobei man Licht einstrahlt, das einen Spektralbereich von mindestens 50 nm umfasst, und wobei mindestens ein Teil der Strahlung im Bereich von 280 nm bis 750 nm liegt, und wobei man mindestens 0.4 Mol Sauerstoff pro Mol Ausgangsverbindung einsetzt.

2. Verfahren nach Anspruch 1, **dadurch gekennzeichnet, dass** man die Bestrahlung in Abwesenheit von Chlor vornimmt, oder dass man die Bestrahlung in Anwesenheit von elementarem Chlor mit Licht einer Wellenlänge von ≥ 280 nm vornimmt, wobei pro Mol CHClF₂ oder CHF₃ maximal 0,50 Mol elementares Chlor im Reaktionsgemisch enthalten ist.

3. Verfahren nach Anspruch 1, **dadurch gekennzeichnet, dass** pro Mol CHClF₂ 0.01 bis 0.50 Mol Chlor enthalten sind.

4. Verfahren nach Anspruch 1, **dadurch gekennzeichnet, dass** pro Mol CHClF₂ oder CHF₃ 0,05 bis 0,20 mol elementares Chlor enthalten sind.

5. Verfahren nach Anspruch 1, **dadurch gekennzeichnet, dass** pro Mol Ausgangsverbindung 0.4 bis 0.9 Mol Sauerstoff eingesetzt werden.

6. Verfahren nach Anspruch 1, **dadurch gekennzeichnet, dass** man die Bestrahlung bei einer Temperatur von 20 bis 300°C durchführt.

7. Verfahren nach Anspruch 1, **dadurch gekennzeichnet, dass** man die Bestrahlung bei einem Druck von 1 bis 11 bar (abs.) durchführt.

8. Verfahren nach Anspruch 1, **dadurch gekennzeichnet, dass** die Reaktanden gasförmig vorliegen.

9. Verfahren nach Anspruch 1, **dadurch gekennzeichnet, dass** die Umsetzung kontinuierlich durchgeführt wird.

10. Verfahren nach Anspruch 9, **dadurch gekennzeichnet, dass** die durchschnittliche Verweilzeit im Reaktor zwischen 0,1 und 3 Minuten liegt.

11. Verfahren nach Anspruch 1, **dadurch gekennzeichnet, dass** man CHClF₂ als Ausgangsverbindung einsetzt.

## Claims

1. Process for preparing C(O)F₂ by photooxidation CHClF₂ or CHF₃ with oxygen, wherein the incident light used encompasses a spectral range of at least 50 nm, and wherein at least some of the radiation is in the range from 280 nm to 750 nm, and wherein at least 0.4 mol of oxygen is used per mole of starting compound.

2. Process according to Claim 1, **characterized in that** the irradiation is undertaken in the absence of chlorine, or **in that** the irradiation is undertaken in the presence of elemental chlorine with light of a wavelength of ≥280 nm, in which case not more than 0.50 mol of elemental chlorine is present in the reaction mixture per mole of CHClF₂ or CHF₃.

3. Process according to Claim 1, **characterized in that** 0.01 to 0.50 mol of chlorine is present per mole of CHClF₂.

4. Process according to Claim 1, **characterized in that** 0.05 to 0.20 mol of elemental chlorine is present per mole of CHClF₂ or CHF₃.

5. Process according to Claim 1, **characterized in that** 0.4 to 0.9 mol of oxygen is used per mole of starting compound.

6. Process according to Claim 1, **characterized in that** the irradiation is carried out at a temperature of 20 to 300°C.

7. Process according to Claim 1, **characterized in that** the irradiation is carried out at a pressure of 1 to 11 bar (abs.).

8. Process according to Claim 1, **characterized in that** the reactants are present in gaseous form.

9. Process according to Claim 1, **characterized in that** the reaction is carried out continuously.

10. Process according to Claim 9, **characterized in that** the average residence time in the reactor is between 0.1 and 3 minutes.

11. Process according to Claim 1, **characterized in that** CHClF₂ is used as the starting compound.

## Revendications

1. Procédé de fabrication de C(O)F₂ par photooxydation de CHClF₂ ou de CHF₃ avec de l'oxygène, une exposition à une lumière qui comprend une plage spectrale d'au moins 50 nm étant réalisée et au moins une partie du rayonnement se trouvant dans la plage allant de 280 nm à 750 nm et au moins 0,4 mole d'oxygène par mole de composé de départ étant utilisé.

2. Procédé selon la revendication 1, **caractérisé en ce que** l'exposition à un rayonnement est réalisée en l'absence de chlore ou **en ce que** l'exposition à un rayonnement est réalisée en présence de chlore élémentaire avec une lumière d'une longueur d'onde ≥ 280 nm, au plus 0,50 mole de chlore élémentaire étant contenu dans le mélange réactionnel par mole de CHClF₂ ou de CHF₃.

3. Procédé selon la revendication 1, **caractérisé en ce que** 0,01 à 0,50 mole de chlore est contenu par mole de CHClF₂.

4. Procédé selon la revendication 1, **caractérisé en ce que** 0,05 à 0,20 mole de chlore élémentaire est contenu par mole de CHClF₂ ou de CHF₃.

5. Procédé selon la revendication 1, **caractérisé en ce que** 0,4 à 0,9 mole d'oxygène est utilisé par mole de composé de départ.

6. Procédé selon la revendication 1, **caractérisé en ce que** l'exposition à un rayonnement est réalisée à une température de 20 à 300°C.

7. Procédé selon la revendication 1, **caractérisé en ce que** l'exposition à un rayonnement est réalisée à une pression de 1 à 11 bar (abs.)

8. Procédé selon la revendication 1, **caractérisé en ce que** les réactifs se présentent sous forme gazeuse.

9. Procédé selon la revendication 1, **caractérisé en ce que** la réaction est réalisée en continu.

10. Procédé selon la revendication 9, **caractérisé en ce que** le temps de séjour moyen dans le réacteur est compris entre 0,1 et 3 minutes.

11. Procédé selon la revendication 1, **caractérisé en ce que** CHClF₂ est utilisé en tant que composé de départ.
